(19) 

Europäisches
Patentamt
European
Patent Office
Office européen
des brevets

(11) **EP 2 196 179 A1**

(12) **EUROPEAN PATENT APPLICATION**
published in accordance with Art. 153(4) EPC

(43) Date of publication:
**16.06.2010 Bulletin 2010/24**

(21) Application number: **08830878.8**

(22) Date of filing: **11.09.2008**

(51) Int Cl.:
*A61K 8/19* (2006.01)      *A61K 8/97* (2006.01)
*A61Q 19/00* (2006.01)

(86) International application number:
**PCT/ES2008/000585**

(87) International publication number:
**WO 2009/034208 (19.03.2009 Gazette 2009/12)**

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**AL BA MK RS**

(30) Priority: **14.09.2007 ES 200702453**

(71) Applicant: **Clayspray, S.l.
20006 San Sebastián (ES)**

(72) Inventor: **GONZÁLEZ SEGURA, Juan Luis
20006 San Sebastián (ES)**

(74) Representative: **Isern-Jara, Nuria
J. Isern Patentes y Marcas
Avda. Diagonal 463 Bis 2°
08036 Barcelona (ES)**

(54) **COSMETIC COMPOSITION IN THE FORM OF A SPRAY FOR TOPICAL ADMINISTRATION**

(57)    Cosmetic composition for external administration in spray form. The present invention refers to a cosmetic composition for external administration in spray form and to a production process of this cosmetic composition containing at least an active compound, a continuous external dispersing phase (natural mineral spring water and/or wine), sodium polyphosphate, sodium polyacrylate and citric acid. The present invention also refers to a dosing and application device for this composition and to the use thereof for the production of a cosmetic composition of external use in spray form.

EP 2 196 179 A1

# Description

## FIELD OF THE INVENTION

The present invention falls within the cosmetic and skin treatment field.

More specifically the present invention refers to a cosmetic composition for its external administration in spray form and to a production process of said cosmetic composition, which at least contains an active compound, a continuous external dispersing phase (natural mineral spring water and/or wine), sodium polyphosphate, sodium polyacrylate and citric acid.

The present invention also refers to a device for dosing said composition and to the use of said cosmetic composition in the manufacture of a cosmetic product intended for cosmetic skin treatment.

## BACKGROUND OF THE INVENTION

The main function of epidermis is to produce the horny stratum (HS), a selectively permeable membrane that protects us against excessive loss of water and chemical and microbial attacks. The HS has three main functions: barrier, mechanical protection and desquamation. The combination of barrier properties and their cell moisturizing capacities are important to maintain skin flexibility.

Among the essential components of HS stand out keratins (specialized proteins from the outer layer of corneocytes), lipids, proteins, natural moisturizing factor, desmosomes (specialized adhesion structures) and enzymes.

HS lipids contribute to keep the water permeability barrier. Among its components stand out ceramides, cholesterol and fatty acids.

The natural moisturizing factor is a mixture of amino acids, amino acids derivatives and specific salts. It occurs only in HS cells and represents 10% of its dry weight. The role of natural moisturizing factor is bound to the fact that its constituents are very water soluble and hygroscopic. The natural moisturizing factor is able to absorb water from the atmosphere, as well as from the skin inside; water is a HS plasticizer, it prevents abnormal skin desquamation and cracking.

The barrier function of HS is very dependent on lipid concentrations and types, as well as on natural moisturizing factor. Without them HS hydration is not effective, what leads to a mechanical failure and the skin loses its properties of softness, elasticity and flexibility.

Clay or mud can help you to have a clean skin without blackheads, pimples or acnes. Natural mud or clay masks are ideal for fatty skins.

In this sense it is fundamental to receive all appropriate treatments assuring the skin care, in order to keep the horny stratum healthy.

For this reason in the present invention a procedure and a cosmetic composition have been designed, which are able to keep the epidermis in perfect condition, regarding hydration as well as stability and elasticity. The inventors of the present invention have developed a cosmetic composition for external use, to be applied in spray form, which at least contains an active compound, natural mineral spring water, sodium polyphosphate, polyacrylate polymer and citric acid, so that its production form together with the election of the spring water and the active compound make her a suitable combination of elements that promotes hydration, firmness and elasticity of the skin.

One of the active compounds selected according to the present invention is clay or mud. In the state of the art it is only too well known that clay has been used from old times for medicinal and embellishment purposes. Pre-columbian cultures of Mexico had used clay for curing and preserving purposes and even today the peasants in north India use clay shampoo.

Clay or mud is one of the most suitable ingredients to clean the skin. Clay in its several forms helps to stimulate circulation in the skin and even more important, when using clay in masks, it dries on the skin surface, absorbing its oils and impurities. When drying, clay texture provides high absorbent power.

The use of the clay in cosmetics is not new. In Europe it has been used in combination with herbs and essential oils to clean and cure the skin. The use of clay is safe and suitable for all skin types, when mixed with other ingredients like for example herbs or oils. Aromatherapy and clay make up a powerful combination.

There are several clay types. Some types are difficult to find. Green clay or French clay is commonly used in cosmetics. This clay can be easily obtained in natural food stores.

However, this type of clay masks presents a series of inconveniences, usually derived from their application way - since they require a long application process and a later cleaning, which usually requires the help of third people for said application - without considering the secondary micro-environmental contaminations associated with the preservation of raw materials.

Therefore, the object of the present invention solves all these inconveniences in a quick and effective way, providing a cosmetic composition that contains natural elements as clay capable of being used by external way in spray form, for a quick, clean and simple application that allows a homogeneous distribution of the same and assures the preservation of the microbiological conditions when being inside a tightly closed container. For this purpose the present invention also shows a production process of this composition as well as its use in cosmetics for moisturizing and reaffirming the skin.

In the state of the art there are documents that describe cosmetic compositions containing some of the elements which are claimed in the present memorandum, however their use is different and the use of clay or polymers is aimed at another applications. For example, the American patent application US 740578 describes cosmetic compositions comprising a non soluble polymer, a film forming polymer and an organophilic clay, but in this case they are used as a make-up base for eyes and the organophilic clays are used as organic solvent (vehicle) and not as an active compound. Furthermore, natural spring water is fundamental in our invention, but in this document there is no mention of any component with such characteristics.

Another document of the state of the art dealing with polymeric particles and clays is the patent EP 95306121, however the matter is in this case detergent compositions that comprise clay flocculating polymers and therefore they are neither used in the skin cosmetic nor less in spray form.

In the state of the art there are also sprayable cosmetic preparations, but only for hair, as described for example in the American patent US 429894, and their composition contains plastic elements like silicone as a hair conditioner.

Other documents from the state of the art like the patents US 3372043, KR 20040020328 and DE19951000447 refer to compositions comprising clay in form of micro- or nanonized powder which are obtained by the chemical method of "Spray-dryer." In said cases this technique does not have anything to do with our invention, because their heterogeneous mixtures are obtained by the method of simultaneous powdering and drying, and it does not mean that these compositions are finally applied in spray form.

**BRIEF DESCRIPTION OF THE INVENTION**

The present invention refers a cosmetic composition for external administration in spray form and to the use of the cosmetic composition in the manufacture of a cosmetic product for a cosmetic skin treatment based on the moisturizing, reaffirming and smoothening properties of the active compounds used in the present invention, such as red or white clays, wine and cocoa.

The present invention also refers to a production process of a cosmetic composition containing at least an active compound, a surrounding liquid dispersing phase like, for example, natural mineral water from spring, sodium polyphosphate, sodium polyacrylate and citric acid.

In the present invention a liquid clay is preferably used as the active compound, capable to be applied in spray form thanks to the production process specified in the present invention.

**DETAILED DESCRIPTION OF THE INVENTION**

Just as used in this description, it should be understood, unless otherwise specified, that the following terms have the next indicated meanings:

"Active compound" is one included in the cosmetic composition that confers to it moisturizing, reaffirming, smoothening properties, etc. Active compounds preferred in the present invention comprise white and/or red clays, wine and cocoa, alone or in combination.

By "natural mineral water coming from spring" it is meant hypoallergenic water without preservatives. This water is collected in a sterile room without human operation, directly from the spring at 100 meters deep in the heart of an aquifer, for example, and without limiting sense, from a protected karstic area declared nature reserve, like the one supplying Elkai Spring. The physical-chemical characteristics of this water are, for example:

- Low mineralization
- Dry residue: 236 mg/l
- pH: 7,75
- Potassium: 0,3 mg/l
- Magnesium: 5,8 mg/l
- Sodium: 6,8 mg/l
- Chlorides: 17,8 mg/l
- Silica: 2,3 mg/l
- Nitrates: 1,4 mg/l
- Calcium: 68,8 mg/l
- Sulfates: 7,7 mg/l
- Bicarbonates: 187,9 mg/l

By "spray" it is meant a container with a special device to pulverize the liquids contained in it, as well as the liquid substance contained in this container or the application form of the composition object of this invention.

An aspect of the invention refers to a production process of a cosmetic composition containing at least an active compound, an external liquid dispersing phase (natural mineral spring water or wine), sodium polyphosphate, polyacrylate polymer and citric acid.

In the present invention a liquid clay is preferably used as the active compound, capable to be applied in spray form thanks to the production process specified in the present invention.

Generally, the process comprises the following steps:

1. *Extraction and milling of the raw material:*

In the mining exploitation, a clay is previously <u>selected</u> in order to ensure its purity and trace elements content, as well as other natural properties. After the raw material selection, the so called product <u>dry milling</u> process is carried out. This process is critical and fundamental to obtain micronized clay of a maximum particle size between 50 microns and 100 microns. This process is vital to finally attain excellent fineness, texture, softness and cosmetic properties, as well as effective interaction at molecular level between clay and the applied formulation, according to our organoleptic requirements.

*2. Physical-chemical emulsion process:*

Starting from the product obtained in the dry milling process, a heterogeneous, polyphasic and uniform emulsion is formed, consisting of:

- a discontinuous and surrounded *internal dispersed phase,* in which the clay is contained;
- a continuous and surrounding *external dispersing phase* of water, in an emulsifying interface that allows a decrease in the interfacial tension of micronized clay particles by means a protective film which is formed by adsorption and makes up a double dielectric layer between the phases, thus preventing emulsion coalescence by rendering it more homogeneous and stable.

The addition of the polymer to the process allows to form a net structure or mesh, the web of which includes the internal phase of the emulsion. In its mesh formed by the external and emulsifying phase are embedded the water drops. The election of the emulsifying characteristics, taking into account that the emulsifier should be less soluble in the internal phase than in the external phase, along with the specific size and granulometry of the micronized clay, is another factor which contributes to attain a stable emulsion.

The specific formulation and composition object of the present invention, based on the selection of the essential products like clay and water, as well as the election of co-emulsifiers like sodium metaphosphate and sodium polyacrylate, along with the consequent corrections with citric acid as a preservative, antioxidant, buffer and chelating agent, provides in general the own specific and singular properties that help to keep epidermis in a perfect condition of hydration, firmness and elasticity.

The material and equipment used in the production are: graduated cylinders, beakers, scales, spatula, stirring system and dosing or packing system.

**Methodology and process for obtaining the formulation:**

The process for obtaining the cosmetic composition according to the present invention comprises the following steps, illustrated by way of specific examples, wherein the active compound is clay and the external dispersing phase is natural spring water, for which reason the steps specified herein do not have a limitative sense, but they are rather exposed by way of specific example of the invention:

a) selecting a clay,
b) dry milling the clay,
c) weighing the components of the necessary products to prepare the whole formulation,
d) measuring the necessary volume of natural spring water,

e) putting the dry milled clay in a container and adding the water while mixing gently with a spatula,
f) adding first the sodium metaphosphate to the mixture obtained in step e), followed by the sodium polyacrylate and lastly the citric acid,
g) beating the mixture in an industrial mixer to 600-800 rpm for 15-20 minutes to form the emulsion,
h) transferring the emulsion formed in the step g) to the packaging hopper,
i) sterilizing the dosed and packed product totally (the technology used for this sterilization is called "sterilization by beta rays or electron bombardment". The sterilization is absolute and the health warranty meets the European regulations),
j) performing the microbiological and bacteriological analyses to certify full sterilization and absence of germs or noxious components according to the legislation and the international cosmetic regulations.

The present invention also refers to a cosmetic composition for its external administration in spray form and to the use of the cosmetic composition for the production of a cosmetic product for cosmetic treatment of the skin, thanks to the moisturizing, reaffirming and smoothening properties of the active compounds used in the present invention, such as red or white clays, wine and cocoa.

Said composition can contain the following components:

*1. Active compound selected from the group formed by clay and cocoa alone or in combination:*

a) Clay:

The liquid clay formulation object of the present invention, commercially called ClaySpray®, is composed of clays based preferably on aluminum silicates with different chemical and mineralogical contents ($Al_2O_3$, $SiO_2$ILL, K, Qz) depending on the clay type, which determine the color of the different components, and has physical-chemical characteristics of pH 6,5 and density 1,2 (g/cm$^3$). After a mill process micronized particles of different size are obtained.

The granulometric features of clay and its selected particle size increase the interfacial surface and along with the emulsifying action allow to form a protective film owing to said factors and resulting in coalescence decrease and bigger stability.

The inherent physical-chemistry structure of clay adds plastic and thixotropic properties to the whole emulsion. For this, one of the most notorious features is that the product does not drip neither does it drain after application on the body, allowing an easy, homogeneous and quick application, what represents a substantial advantage with regard to other external administrations.

Up to now, the inventors of the present invention have determined the interactions taking place between clays, water and organic molecules, all of them being reversible interactions. That is to say, if we subject the enclosed solid to thermal or physical-chemical treatment, the material will recover its original form so many times the cycle is repeated, as it happens with an accordion that expands and contracts according to the force being applied to its bellows. In this aspect clays are similar.

Consequently, we have determined that clays play an important role in multiple fields, provided the dimensions of their interlaminar space are permanent, that is to say, that the interlaminar separation remains constant, independently of which treatments the material is subjected to. Hence, clays can act as true catalysts in a great number of reactions of industrial interest, for what scientists devised the way to make them stable by fixing their interlaminar distance. The strategy that would allow to achieve this objective was set in 1974 during a meeting of specialists in catalysis. After several discussions it was decided to proceed as follows: in order to prevent the clay laminar building from recovering its initial form it is necessary to bolster the space between the layers by means of pillars. Therefore, when using clays it is necessary to develop chemical species of appropriate size, capable of penetrating the interlaminar space of clay flakes and expanding their structure, and later on forming another stable phase that resists disintegration (pillaring concept).

b) Cocoa:

The internal phase of the emulsion process is replaced with pure cocoa powder, without mixtures, using the specific formulations containing the components investigated for the production process of the liquid clay.

c) Clay with cocoa:

The internal phase of the emulsion process is replaced with cocoa powder and red clay, using the specific formulations containing the components investigated for the production process of the liquid clay.

2. *Continuous surrounding external liquid dispersing phase selected of the group formed by natural mineral water and red wine, alone or in combination:*

1. Water:

The selection of water characteristics for the aqueous phase is fundamental for the final result of all our products. By using a natural mineral water of middle pH (pH 7,75 approximately), weak mineralization and consequently low residue we achieve higher dispersion of the clay micronized particles enclosed in the emulsifier, as well as control and stability of the whole industrial production process.

The water is hypoallergenic and free of preservatives. This water is collected in a sterile room without human operation, directly from the spring at 100 meters deep in the heart of an aquifer from a protected karstic area declared nature reserve, like the one supplying Elkai Spring.

*CHEMICAL PHYSICAL ANALYSIS*

- Low mineralization
- Dry residue: 236 mg/l
- pH: 7,75
- Potassium: 0,3 mg/l
- Magnesium: 5,8 mg/l
- Sodium: 6,8 mg/l
- Chlorides: 17,8 mg/l
- Silica: 2,3 mg/l
- Nitrates: 1,4 mg/l
- Calcium: 68,8 mg/l
- Sulfates: 7,7 mg/l
- Bicarbonates: 187,9 mg/l

2. Wine:

The constituent water of the continuous and surrounding external dispersing phase is replaced by wine (red wine).

3. Sodium metaphosphate:

Synonyms: sodium hexametaphosphate, sodium tetrapoly-phosphate, Graham salt, vitreous sodium polyphosphates, sodium polymetaphosphates.

Chemical formula: heterogeneous mixtures of sodium salts of linear condensed polyphosphoric acids of general formula:

$$H_{(n+2)}P_nO_{(3n+1)},$$

where n: is equal or higher than 2.

Sodium metaphosphate acts as a thickening, sequestering/ chelating, stabilizing, co-emulsifying and antiflocculating surfactant agent, increasing adhesiveness and cohesiveness between particles, due to its agglutinating properties.

It increases the hydrophobic power forming a film and making up a double dielectric layer between the phases which prevents emulsion coalescence and sedimentation by rendering it more homogeneous and stable.

4. Polymer: sodium polyacrylate

Synonyms: polyacrylate, acrylamide polymer, acrylamide-acrylate polymer.

Reticulated sodium polyacrylate (monomer:

-CH$_2$-CH(CO$_2$Na)-) is a sodium salt of reticulated acrylic acid polymer.

Sodium polyacrylate is a polymer that contains many acrylate monomers bonded end-to-end in a big chain. The reticulation between chains "ties" them together.

Sodium polyacrylate is partially neutralized. That means that everywhere starting from 50-70% of the groups COOH (the acid groups) has been converted into the respective sodium salt.

The clay object of the present invention with registered mark ClaySpray® is enriched with macromolecular hydrogels (sodium polyacrylate), which are considered as the new intelligent materials of this millennium, able to retain up to 800 times their weight in water, resulting in a very effective vehicle technology as for enhancing the moisturizing, reaffirming and tensile effects of the active compounds (mud or clay and cocoa) and preserving for months the product properties and its original humidity, whether in spray or in non hermetic containers.

Properties:

It also increases water-repellent power and film forming properties. The molecular weight is higher and the absorbing capacity increases up to 800 times its weight in water.

It provides an appropriate viscosity level which improves emulsion spreadability and gliding.

The addition of sodium polyacrylate prevents the soaping effect of the emulsion (antifoaming). It makes the product emollient, soft and shining and avoids cold feeling caused by evaporation.

It confers to the formulation thixotropic, surfactant, humectant and rheological properties. It confers plasticity to the product, providing a plastic flow of constant viscosity, and increases adhesiveness and cohesiveness between particles due to its deflocculating and agglutinating properties (as it lowers of the particle coalescence and increases the interfacial area).

5. Citric acid:

Citric acid is an organic tricarboxylic acid that exists in most fruits, mainly in citrus fruits like lemon and orange. Its chemical formula is C$_6$H$_8$O$_7$ (3-hydroxy-1,3,5-pentano-tricarboxylic acid).

Properties:

Antioxidant, preservative, buffering and stabilizing additive for the emulsion. It modifies and controls the final product pH giving a pH neutral for skin (pH of approx. 5,5-6) in all our products.

Thanks to the production process object of the present invention, by applying the investigation results of recent years a stable and microbiological pure emulsion is obtained, as previously mentioned.

Specific formulations have been developed for each product, all them in form of liquid emulsion ready for use.

Preferred formulations of the present invention are those containing clay as compound active, natural spring water as continuous external dispersing phase, sodium polyphosphate, sodium polyacrylate polymer and citric acid.

Another preferred formulation is that formed by cocoa as active compound, natural spring water as continuous external dispersing phase, sodium polyphosphate, sodium polyacrylate polymer and citric acid.

Another preferred formulation is that formed by cocoa with clay as active compound, natural spring water as continuous external dispersing phase, sodium polyphosphate, sodium polyacrylate polymer and citric acid.

Another preferred formulation is that formed by cocoa with clay as active compounds, natural spring water as continuous external dispersing phase, sodium polyphosphate, sodium polyacrylate polymer and citric acid.

Another preferred formulation is that formed by clay as active compound, red wine as continuous external dispersing phase, sodium polyphosphate, sodium polyacrylate polymer and citric acid.

Another preferred formulation is that formed by cocoa as active compound, red wine as continuous external dispersing phase, sodium polyphosphate, sodium polyacrylate polymer and citric acid.

Another preferred formulation is that formed by cocoa with clay as active compound, red wine as continuous external dispersing phase, sodium polyphosphate, sodium polyacrylate polymer and citric acid.

Thanks to its absorbing capacity and antiseptic properties the cosmetic formulation object of the present invention extracts by means of the active components and the external phase the toxins accumulated under the skin and eliminates skin impurities. Furthermore, it possesses vasoconstrictor and antiinflammatory qualities.

When applying the formulation, finely distributed over the skin by means of the spray, useful substances penetrate in the body and toxins, poisons and disturbing agents are eliminated and absorbed by the formulation (mud), and skin functions are also stimulated.

The formulation object of the present invention uses clay in all its product range as a vehicle that transmits the clay properties for the extraction of toxins, thus enabling other active substances, like the above mentioned and other now being investigated, to penetrate and display their properties.

Container specifications (Spray):

The reported characteristics of liquid clay as regarding its long-term stability and drying up resistance, when hermetically packed to avoid biological contamination, allow for its use at any moment, without previous elaboration, thanks to an advantageous recyclable double chamber aluminum monoblock container.

At present this is a very innovative system at international level, since the company ClaySpray is the first manufacturer in the market that uses a spray container to pack formulations with liquid clay.

To ensure its purity, the product is always isolated in a tight chamber without any contact with the propeller gas.

Nitrogen, an inert and ecological gas, respectful with the product, that preserves at any moment its microbiologic purity and freshness, is used as the propellant, making unnecessary to blend the product with preservatives o gas.

According to a first important aspect the invention refers to a liquid cosmetic composition for external administration in spray form, which at least contains:

- an active compound selected from the group formed by clay and cocoa and/or their derivatives, alone or in combination,
- a continuous external dispersing phase selected from the group formed by natural mineral spring water and wine, alone or in combination,
- sodium polyphosphate,
- sodium polyacrylate and,
- citric acid.

According to another aspect, the clay of the liquid cosmetic composition for external administration in spray form is composed of aluminum silicates.

According to another aspect, natural mineral water in the liquid cosmetic composition for external administration in spray form has a middle pH of 7,75 and following physical-chemical characteristics:

- Low mineralization
- Dry residue: 236 mg/l
- Potassium: 0,3 mg/l
- Magnesium: 5,8 mg/l
- Sodium: 6,8 mg/l
- Chlorides: 17,8 mg/l
- Silica: 2,3 mg/l
- Nitrates: 1,4 mg/l
- Calcium: 68,8 mg/l
- Sulfates: 7,7 mg/l
- Bicarbonates: 187,9 mg/l

According to another aspect, the liquid cosmetic composition for external administration in spray form contains sodium polyphosphate of the general formula:

$$H_{(n+2)}P_nO_{(3n+1)},$$

where n: is equal or higher than 2.

According to another aspect, the liquid cosmetic composition for external administration in spray form has a neutral pH value between 5,5 and 6.

A second important aspect of the present invention is a process for the preparation of a liquid cosmetic composition for external administration in spray form comprising the following steps:

a) selecting an active compound,
b) dry milling the active compound,
c) weighing the components of the necessary products to prepare the whole formulation,
d) measuring the necessary volume of surrounding external liquid dispersing phase,
e) putting the dry milled active compound in a container and adding the external liquid phase while mixing gently with a spatula,
f) adding the sodium metaphosphate to the mixture obtained in step e), followed by the sodium polyacrylate and lastly the citric acid,
g) beating the mixture in an industrial mixer to 600-800 rpm for 15-20 minutes to form the emulsion,
h) transferring the emulsion formed in the step g) to the packaging hopper,
i) sterilizing the dosed and packed product totally, and
j) performing the microbiological and bacteriological analyses to certify full sterilization and absence of germs or noxious components according to the legislation and the international cosmetic regulations.

According to another aspect, the active compound obtained after dry milling has a particle size between 50-100 microns.

According to another aspect, the sterilization of the dosed and packed product is carried out by means of beta rays or electron bombardment.

According to a third aspect, the present invention refers to a dosage and application device of the cosmetic composition which it is characterized for being a recyclable double chamber aluminum monoblock container, wherein the cosmetic composition is isolated in a tight chamber without contact with the propellant and the used propellant is nitrogen.

A fourth important aspect of the present invention is the use of the composition as defined in the description for the preparation of a cosmetic composition for external use in form of a tightly closed spray that ensures the microbiologic stability of said composition.

Claims

1. Liquid cosmetic composition for external administration in spray form,
characterized in that:

- it contains at least an active compound selected from the group formed by clay and cocoa and/or their derivatives, alone or in combination,
- a continuous external dispersing phase selected from the group formed by natural mineral spring water and wine, alone or in combination,
- sodium polyphosphate,
- sodium polyacrylate and,
- citric acid.

2. Liquid cosmetic composition for external administration in spray form according to claim 1, **characterized in that** the composition contains preferably clay as active compound, natural mineral spring water as continuous external dispersing phase, sodium polyphosphate, sodium polyacrylate and citric acid.

3. Liquid cosmetic composition for external administration in spray form according to claim 2, **characterized in that** the clay is composed of aluminum silicates.

4. Liquid cosmetic composition for external administration in spray form according to claim 2, **characterized in that** the natural mineral water has a middle pH of 7,75.

5. Liquid cosmetic composition for external administration in spray form according to any of the claims 2 and 3, **characterized in that** the natural mineral water has the following physical-chemical characteristics:

   - Low mineralization
   - Dry residue: 236 mg/l
   - Potassium: 0,3 mg/l
   - Magnesium: 5,8 mg/l
   - Sodium: 6,8 mg/l
   - Chlorides: 17,8 mg/l
   - Silica: 2,3 mg/l
   - Nitrates: 1,4 mg/l
   - Calcium: 68,8 mg/l
   - Sulfates: 7,7 mg/l
   - Bicarbonates: 187,9 mg/l

6. Liquid cosmetic composition for external administration in spray form according to any of the claims 1 and 2, **characterized in that** the sodium polyphosphate has the general formula:

$$H_{(n+2)}P_nO_{(3n+1)},$$

where n: is equal or higher than 2.

7. Liquid cosmetic composition for external administration in spray form according to the preceding claims, **characterized in that** the pH of the composition has a neutral value between 5,5 and 6.

8. Liquid cosmetic composition for external administration in spray form according to the preceding claims, **characterized in that** it is packed inside a tightly closed container that ensures the microbiologic stability of the composition.

9. Process for the preparation of a liquid cosmetic composition for external administration in spray form as defined in the claim 1, **characterized in that** it comprises the following steps:

   a) selecting an active compound,
   b) dry milling the active compound,
   c) weighing the components of the necessary products to prepare the whole formulation,
   d) measuring the necessary volume of surrounding external liquid dispersing phase,
   e) putting the dry milled active compound in a container and adding the external liquid phase while mixing gently with a spatula,
   f) adding the sodium metaphosphate to the mixture obtained in step e), followed by the sodium polyacrylate and lastly the citric acid,
   g) beating the mixture in an industrial mixer to 600-800 rpm for 15-20 minutes to form the emulsion,
   h) transferring the emulsion formed in the step g) to the packaging hopper,
   i) sterilizing the dosed and packed product totally, and
   j) performing the microbiological and bacteriological analyses to certify full sterilization and absence of germs or noxious components according to the legislation and the international cosmetic regulations.

10. Process for the preparation of a liquid cosmetic composition for external administration in spray form according to the claim 9, **characterized in that** the active compound obtained after the dry milling has a particle size between 50-100 microns.

11. Process for the preparation of a liquid cosmetic composition for external administration in spray form according to the claim 9, **characterized in that** the sterilization of the dosed and packed product is carried out by means of beta rays or electron bombardment.

12. Dosage and application device of the cosmetic composition of claim 1, **characterized in that** it is a recyclable double chamber aluminum monoblock container.

13. Dosage and application device of the cosmetic composition of claim 12, **characterized in that** the cosmetic composition is isolated in a tight chamber without contact with the propellant.

14. Dosage and application device of the cosmetic composition of claim 13, **characterized in that** nitrogen is used as the propellant.

15. Use of the composition as defined in the claims 1 to 8 for the preparation of a cosmetic composition.

16. Use of the composition as defined in the claims 1 to 8 for the preparation of a cosmetic composition for external use in spray form.

# INTERNATIONAL SEARCH REPORT

| International application No. |
| --- |
| PCT/ ES 2008/000585 |

**A. CLASSIFICATION OF SUBJECT MATTER**

see extra sheet

According to International Patent Classification (IPC) or to both national classification and IPC

**B. FIELDS SEARCHED**

Minimum documentation searched (classification system followed by classification symbols)

A61K, A61Q

Documentation searched other than minimum documentation to the extent that such documents are included in the fields searched

Electronic data base consulted during the international search (name of data base and, where practicable, search terms used)

INVENES, EPODOC, WPI

**C. DOCUMENTS CONSIDERED TO BE  RELEVANT**

| Category* | Citation of document, with indication, where appropriate, of the relevant passages | Relevant to  claim No. |
| --- | --- | --- |
| A | US 2007286834 A (POPOV A G) 13.12.2007. claim 10. | 1 |
| A | KR 20060102847 A (KIM N J) 28.09.2006. Abstract. | 1 |
| A | WO 9956720 A1 (THE PROCTER & GAMBLE COMPANY) 11.11.1999. the whole document. | 1 |
| A | ES 2082515 T3 (THE BOOTS COMPANY PLC) 16.03.1996. the whole document. | 1 |

☐ Further documents are listed in the continuation of Box C.     ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
| --- | --- |
| 28.November.2008          (28.11.2008) | (09/01/2009) |
| Name and mailing address of the ISA/ O.E.P.M. Paseo de la Castellana, 75 28071 Madrid, España. Facsimile No.   34 91 3495304 | Authorized officer  A. Amaro Roldán  Telephone No. +34 91 349 84 13 |

Form PCT/ISA/210 (second sheet) (July 2008)

## INTERNATIONAL SEARCH REPORT

Information on patent family members

International application No.

PCT/ ES 2008/000585

| Patent document cited in the search report | Publication date | Patent family member(s) | Publication date |
|---|---|---|---|
| US 2007286834 A | 13.12.2007 | NONE | ------------ |
| KR 20060102847 A | 28.09.2006 | NONE | ------------ |
| WO 9956720 A | 11.11.1999 | AU 7293398 A<br>JP 2000516643 T | 23.11.1999<br>12.12.2000 |
| ES 2082515 T3 | 16.03.1996 | WO 9307855 A<br>AU 2774292 A<br>EP 0609301 A<br>AT 133066 T<br>DE 69207777 T | 29.04.1993<br>21.05.1993<br>15.02.1996<br>15.02.1996<br>23.05.1996 |

Form PCT/ISA/210 (patent family annex) (July 2008)

## INTERNATIONAL SEARCH REPORT

International application No.

PCT/ ES 2008/000585

CLASSIFICATION OF SUBJECT MATTER

*A61K 8/19* (2006.01)
*A61K 8/97* (2006.01)
*A61Q 19/00* (2006.01)

Form PCT/ISA/210 (extra sheeet) (July 2008)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 740578 A
- EP 95306121 A
- US 429894 A
- US 3372043 A
- KR 20040020328
- DE 19951000447